(19) Europäisches Patentamt
European Patent Office
Office européen des brevets

(11) EP 4 342 470 A1

(12) **EUROPEAN PATENT APPLICATION**
published in accordance with Art. 153(4) EPC

(43) Date of publication:
**27.03.2024 Bulletin 2024/13**

(21) Application number: **22804985.4**

(22) Date of filing: **18.05.2022**

(51) International Patent Classification (IPC):
***A61K 31/496*** *(2006.01)* ***A61P 35/00*** *(2006.01)*

(52) Cooperative Patent Classification (CPC):
**A61K 31/496; A61P 35/00**

(86) International application number:
**PCT/KR2022/007115**

(87) International publication number:
**WO 2022/245131 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.05.2021 KR 20210064278**

(71) Applicant: **Onconic Therapeutics Inc.**
**Gangnam-gu**
**Seoul 06236 (KR)**

(72) Inventors:
- **CHA, Hyun Ju**
  **Seoul 06714 (KR)**
- **LEE, Chang Seok**
  **Yongin-si, Gyeonggi-do 16846 (KR)**
- **HAN, Sang Woo**
  **Seoul 06274 (KR)**
- **KIM, John**
  **Seoul 06732 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **PARP INHIBITOR-RESISTANT CANCER THERAPEUTIC AGENT**

(57) The present invention relates to a pharmaceutical composition for treating or preventing a patient with solid cancer resistant to a PARP inhibitor. The pharmaceutical composition according to the present invention can effectively reduce tumor size in a patient with resistance to a PARP inhibitor.

【Figure 4】

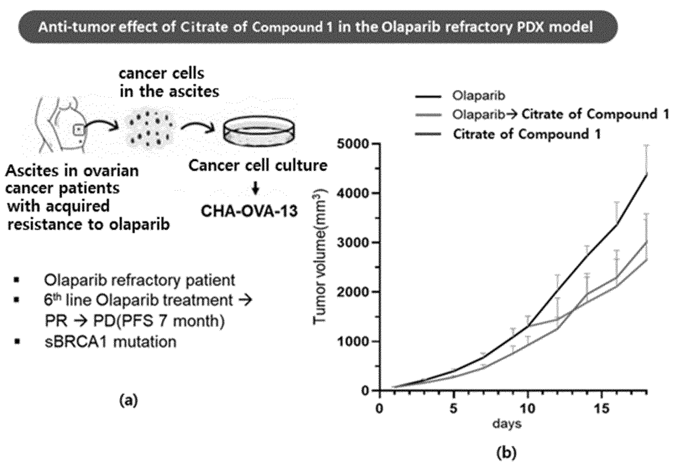


EP 4 342 470 A1

## Description

[Technical Field]

This application claims the benefit of priority based on Korean Patent Application No. 10-2021-0064278 filed on May 18, 2021, the entire contents of which are incorporated herein as part of the present specification.

The present invention relates to a cancer therapeutic agent that can be used for the treatment of a patient with solid cancer resistant to a PARP inhibitor.

[Background Art]

Accumulation of DNA mutations is known as one of the representative causes of cancer. Mammals grow and develop from a single cell, a fertilized egg, through an endless process of cell division, and in this process, mutations inevitably occur in DNA (hereinafter referred to as "DNA damage"). However, DNA damage is repaired by various DNA repair mechanisms such as Homologous Recombination (HR) or Non-Homologous End Joining (NHEJ). Various types of proteins are involved in each DNA repair mechanism, and if mutations occur in some of these proteins, problems occur in the DNA repair mechanism thereby increasing the probability of cancer by several to hundreds of times. In general, when homologous recombination function is lost, the genome becomes unstable, which induces various genetic changes and eventually causes tumors.

BRCA1/2 genes involved in the repair of damaged DNA are genes that suppress tumorigenesis. It is known that when a mutation occurs in the BRCA1/2 gene and its function is reduced, the damaged DNA is not properly repaired and DNA damage accumulates, causing cancer. This is called Homologous Recombination Deficiency (HRD). Breast and ovarian cancers associated with BRCA1/2 gene mutations are well known as homologous recombination deficit tumors. In particular, it is known that the probability of developing breast or ovarian cancer increases by up to 80% and 60% in the case of women with BRCA1/2 gene mutations, respectively. However, BRCA1/2 gene mutation is known to be associated with not only the aforementioned breast and ovarian cancers, but also gastric, pancreatic, prostate, gallbladder, biliary tract, and colorectal cancers.

Poly(ADP-Ribose) Polymerase (PARP) protein is a protein necessary for repairing errors that inevitably occur during DNA replication, and is an enzyme that is activated by recognizing damaged DNA in the nucleus and then activates DNA repair related proteins through a post-translation process (PARrylation). Although 17 PARP families have been known so far, only PARP1/2 has been identified as a DNA repair enzyme having poly(ADP-ribosylation) activity, and is known as an essential enzyme for cell survival.

Homologous Recombination Deficiency tumor is known to show a sensitive response to DNA damage caused by PARP inhibitors. Therefore, PARP inhibitors have great potential in clinical practice as cancer therapeutics. In fact, PARP inhibitors such as olaparib(Lynparza™), rucaparib(Rubraca™), niraparib(ZEJULA™), and talazoparib(Talzenna™) are being prescribed for patients with ovarian, breast or prostate cancer who genetically have the BRCA1/2 mutation (germ-line mutation). In particular, niraparib is used as an agent for maintenance therapy for recurrent epithelial ovarian cancer, highly serous ovarian cancer (including fallopian tubal cancer or primary peritoneal cancer), and the like that fully or partially respond to platinum-based anticancer chemotherapy.

6-{4-[(5-oxo-1,2,3,4,5,6-hexahydrobenzo[h][1,6]naphthyridin-8-yl)methyl]piperazin-1-yl}nicotinonitrile developed as a PARP inhibitor has the structure of Formula I below. The compound of Formula I below or a pharmaceutically acceptable salt thereof exhibits inhibitory activity against not only PARP1/2 but also tankyrase 1/2. Tankyrase is known to be involved in mitosis, which is highly related to the Wnt/β-catenin signaling pathway, DNA repair process, and cell cycle. In addition, tankyrase 1/2 ADP-ribosylates TRF-1 to function as a positive regulator of telomere length, allowing telomere elongation by telomerase. In addition, the compound of Formula I below or a pharmaceutically acceptable salt thereof is expected to have a therapeutic effect on recurrent epithelial ovarian cancer, highly serous ovarian cancer, etc. that fully or partially respond to platinum-based anticancer chemotherapy.

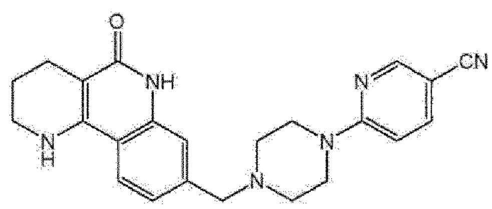

Apart from the potential or expectation of various PARP inhibitors as target therapeutics for cancer treatment, PARP inhibitors including olaparib have high congenital/acquired resistance or refractory rates like other anticancer

drugs. As the ratio of drug resistance to Homologous Recombination Deficiency tumor increases, research on this is being conducted, but no significant progress has been made so far.

In addition, it is not known whether the compound of Formula I can treat solid cancer resistant to other anticancer drugs other than Formula I, particularly solid cancer resistant to PARP inhibitors used in existing standard treatments.

Under this background, the inventors of the present invention studied anticancer agents that can be used in the treatment of patients showing resistance to PARP inhibitors from various angles. As a result, the present invention was completed by confirming that the compound of Formula I of the present invention reduces the tumor size of patients resistant to existing PARP inhibitors (such as olaparib).

[Prior Art Documents]

[Patent Documents]

1. Korean Patent Registration No. 10-1136702(Issue Date: 2012. 4. 20.)
2. Korean Patent Registration No. 10-1146806(Issue Date: 2012. 5. 22.)
3. Korean Patent Registration No. 10-1837047(Issue Date: 2018. 3. 09.)

[Disclosure]

[Technical Problem]

It is an object of the present invention to provide a pharmaceutical composition comprising 6-{4-[(5-oxo-1,2,3,4,5,6-hexahydrobenzo[h] [1,6]naphthyridin-8-yl)methyl]piperazin-1-yl}nicotinonitrile or a pharmaceutically acceptable salt thereof as a composition for the treatment of solid cancer in a patient resistant to PARP inhibitors.

It is another object of the present invention to provide a method for treating solid cancer of a subject by administering 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof to the subject having resistance to PARP inhibitors.

It is still another object of the present invention to provide 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for use in the treatment of solid cancer in patients resistant to PARP inhibitors.

[Technical Solution]

In order to achieve the above object, the present invention provides a pharmaceutical composition comprising 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for treating solid cancer in patients resistant to PARP inhibitors.

In one embodiment of the present invention, the PARP inhibitor may be at least one selected from olaparib, rucaparib, niraparib, and talazoparib, but is not limited thereto.

In one embodiment of the present invention, the patient may have BRCA1/2 mutation.

In one embodiment of the present invention, the patient may have germline BRCA1/2 mutation.

In one embodiment of the present invention, the patient may have somatic BRCA1/2 mutation.

In one embodiment of the present invention, the patient may be a patient who has BRCA1/2 mutation, and initially responded to PARP inhibitors, but acquired resistance during treatment and did not respond to PARP inhibitors or whose cancer recurred.

In one embodiment of the present invention, the patient may be a patient with BRCA1/2 mutation who did not respond to PARP inhibitors.

In one embodiment of the present invention, the patient may be a patient who does not have a BRCA1/2 mutation and have not previously responded to PARP inhibitors or whose cancer recurred.

In one embodiment of the present invention, the solid cancer may be ovarian cancer, breast cancer, prostate cancer, pancreatic cancer, colon cancer, gallbladder cancer, biliary tract cancer, or stomach cancer known to be caused by BRCA1/2 mutation, but is not limited thereto.

In one embodiment of the present invention, the solid cancer may be in the form of progressive solid cancer, recurrent solid cancer or metastatic solid cancer.

In one embodiment of the present invention, if the solid cancer is ovarian cancer, it can be progressive ovarian cancer, recurrent ovarian cancer, high-grade serous ovarian cancer(including fallopian tubal cancer or primary peritoneal cancer), and in the case of metastatic cancer whose primary cancer is ovarian cancer, it may be breast cancer, prostate

cancer, pancreatic cancer, colon cancer, gallbladder cancer, biliary tract cancer, gastric cancer, liver cancer, or lung cancer, but is not limited thereto.

In one embodiment of the present invention, the pharmaceutically acceptable salt of 6-{4-[(5-oxo-1,2,3,4,5,6-hexahydrobenzo[h][1,6]naphthyridin-8-yl)methyl]piperazin-1-yl}nicotinonitrile may be citrate.

In addition, the present invention provides 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for the treatment of a patient with solid cancer resistant to PARP inhibitors.

In addition, the present invention provides a method for treating a patient with solid cancer resistant to a PARP inhibitor by administering an effective amount of 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof.

In addition, the present invention provides an use of 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for the manufacture of a drug for the treatment of a patient with solid cancer resistant to a PARP inhibitor.

[Advantageous Effects]

Since the pharmaceutical composition according to the present invention comprising 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof can effectively reduce the tumor size in a patient with solid cancer resistant to PARP inhibitors, it can be usefully used for the treatment of a patient with solid cancer resistant to PARP inhibitors.

[Description of Drawings]

FIG. 1 is a graph showing the results of analyzing the anticancer effect of the citrate of the compound of Formula I of the present invention (Example 2),
FIG. 2 is a graph showing the experimental results of inhibiting the Wnt signaling pathway activation of the citrate of the compound of Formula I of the present invention (Example 4),
FIG. 3 is a drawing showing the anticancer effect of the citrate of the compound of Formula I of the present invention evaluated using a Xenograft model (Example 5), and
FIG. 4 is a drawing showing the anticancer effect of the citrate of the compound of Formula I of the present invention evaluated using a Xenograft model (Example 6).

[Best Mode]

Hereinafter, the present invention will be described in detail.

In describing and claiming particular features of the present disclosure, the following terms will be used in accordance with the definitions set forth below unless otherwise specified.

It should be understood that although certain aspects herein are described in conjunction with the term "comprising of", other similar aspects described in terms of "consisting of" and/or "consisting essentially of" are also provided.

The term "pharmaceutically acceptable" means a substance that is acceptable to patients from a pharmacological/toxicological point of view in terms of composition, formulation, safety, and the like, and "pharmaceutically acceptable carrier" refers to a medium that does not interfere with the effect of the biological activity of the active ingredient(s) and is non-toxic to the subject upon administration.

The term "resistance" refers to a case in which a drug does not have a desired response (anticancer effect). Specifically, in the present invention, it means to cover all cases where the drug does not respond from the beginning (refractoriness), and where relapse occurs from a certain point after initially responding to the drug(cases where the cancer lesion size decreases at first, then the cancer recurs and increases in size; acquired resistance), despite the PARP inhibitor standard therapy. Herein, "resistance" and "tolerance" may be used interchangeably.

The term "patient" or "subject" or "individual" refers to an organism suffering from a condition whose disease can be treated by administration of the pharmaceutical composition of the present invention, such as solid cancer, and includes both humans and animals. Examples of the subject include, but are not limited to, mammals (e.g., mice, monkeys, horses, cows, pigs, dogs, cats, etc.), and are preferably humans. In addition, "patient" or "subject" or "individual" in the present invention includes a patient with solid cancer that is resistant to PARP inhibitors.

The term "BRCA1/2 mutation" refers to a mutation of BRCA1 and/or BRCA2, and refers to a naturally occurring

mutation at one or more sites of the BRCA1 and BRCA2 genes. Therefore, a mutation may occur in any one gene selected from BRCA1 and BRCA2 genes, or a mutation may occur in both genes, and the mutation may occur in one site or two or more sites of each gene.

As mentioned above, PARP inhibitors have shown great potential in clinical practice as targeted therapy for Homologous Recombination Deficiency tumors, but are known to have a high rate of acquisition of congenital or acquired resistance. There are various mechanisms explaining the reason as follows: i) increase in drug efflux by increasing ABC transporter, ii) activation of PAR chain, iii) reactivation of homologous recombination mechanism by mutation of tumor suppressor genes such as p53 acting on homologous recombination mechanism, iv) stabilization or protection of parts of the replication fork, and v) activation of the Wnt signaling pathway.

Regarding the mechanism of iii) above, it is known that various proteins are involved in the homologous recombination process, and in cancer patients with BRCA1/2 mutations, mutations in other proteins involved in the process of homologous recombination, such as p53, ATM, ATR, and p51, are also found at the same time. Therefore, it has been explained that they may be related to the acquisition of resistance to PARP inhibitors.

Although PARP inhibitors act as specific targeted therapies for patients with homologous recombination deficiency tumors, they have the characteristics of easy acquisition of resistance. Therefore, there is an increasing demand for novel anticancer drugs that can be used for the treatment of patients resistant to PARP inhibitors.

Multidrug resistance (MDR), one of the causes of failure of anticancer treatment, has recently emerged as an important problem in the field of anticancer treatment. This ability is due to the presence of the MDR gene in cancer cells. The MDR1 (ABCB1) gene is a gene that makes a substance called P-glycoprotein (hereinafter referred to as P-gp). P-gp is an enzyme that helps various kinds of drugs to pass through the cell membrane and plays a role in excreting them from the inside of the cell. When many anticancer drugs are continuously administered to patients, drug resistance appears, and overexpression of P-gp is one of the resistance mechanisms. Therefore, when P-gp is overexpressed, drugs that are substrates of P-gp are excreted out of the cell by P-gp, and thus do not exhibit drug efficacy. In the use of anticancer drugs, this multi-drug resistance acts as an important limiting factor, and various studies are being conducted to overcome this multi-drug resistance. As such, cancer cells in which P-gp is overexpressed can suppress P-gp function or overcome resistance by selecting anticancer drugs that are not used as P-gp substrates.

The citrate of the compound of Formula I of the present invention is confirmed to have a significantly lower efflux ratio by P-gp compared to conventional PARP inhibitors. Therefore, it can be usefully used for the treatment of patients resistant to PARP inhibitors.

The Wnt signaling pathway has been implicated in embryonic development, tissue homeostasis and various diseases. Overactive signaling causes accumulation of β-catenin, which translocates into the nucleus and promotes transcription of oncogenes and cell growth. Accordingly, efforts are being made to develop therapeutic agents that block the Wnt signaling pathway.

Recent studies have reported that the resistance mechanism of PARP inhibitors is related to the Wnt signaling pathway. That is, PARP inhibitors activate the Wnt signaling pathway, and it is known that resistance occurs by activation of the Wnt signaling pathway. Therefore, resistance to PARP inhibitors can be overcome by selecting anticancer drugs that can block the Wnt signaling pathway in cancer cells that have acquired resistance to PARP inhibitors.

The citrate of the compound of Formula I of the present invention was found to inhibit the Wnt signaling pathway. Therefore, it can be usefully used for the treatment of patients resistant to PARP inhibitors.

In the present invention, it was found that when a patient with solid cancer resistant to PARP inhibitor was treated with 6-{4-[(5-oxo-1,2,3,4,5,6-hexahydrobenzo[h][1,6]naphthyridin-8-yl)methyl]piperazin-1-yl}nicotinonitrile(Formula I compound) or a pharmaceutically acceptable salt thereof, the size of the solid cancer was reduced.

This fact can be confirmed through, for example, a test in which cell lines resistant to PARP inhibitors(olaparib, rucaparib, niraparib, or talazoparib) or primary cells(CHA-OVA-13 cell) isolated from cancer(e.g.: ovarian cancer) patients resistant to PARP inhibitors are treated with a Formula I compound or a pharmaceutically acceptable salt thereof. Specifically, after selecting a cell line resistant to PARP inhibitors among BRCA mutation-positive ovarian cancer or breast cancer cell lines such as HCC1937, SNU-251, BT474 and SNU-119(e.g.: $IC_{50}$ value is 50 uM or higher), it can be confirmed that cell death occurs through a test in which the cell line is treated with the compound of Formula I or a pharmaceutically acceptable salt thereof.

In addition, the anticancer activity mechanism of the compound of Formula I can be confirmed through an experiment to confirm the expression level of proteins related to apoptosis, homologous recombination, and signal transduction such as pATR, pCHK1, pAKT, tankyrase, cleaved caspase 3, cleaved PARP protein in PARP inhibitor-resistant cell lines where apoptosis occurs by treatment with the compound of Formula I or a pharmaceutically acceptable salt thereof.

This mechanism can also be confirmed by the results of clinical trials targeting animal models transplanted with PARP inhibitor-resistant cell lines and actual PARP inhibitor-resistant cancer patients.

The present invention provides a pharmaceutical composition containing the compound represented by Formula I below, "6-{4-[(5-oxo-1,2,3,4,5,6-hexahydrobenzo[h][1,6]naphthyridin-8-yl)methyl]piperazin-1-yl}nicotinonitrile" or a

pharmaceutically acceptable salt thereof for the treatment of solid cancer in patients with resistance to PARP inhibitors.

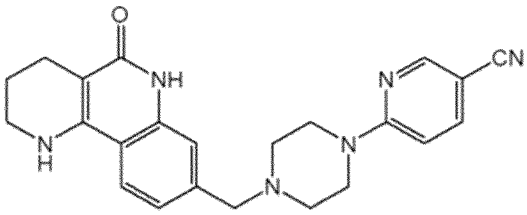

Since the compound of Formula I shows inhibitory activity against PARP1/2, it can be used as a target treatment for patients with Homologous Recombination Deficiency tumor and is an anticancer agent capable of inhibiting tankyrase 1/2 at the same time.

Tankyrase is involved in telomere homeostasis, Wnt/β-catenin signaling, glucose metabolism, and cell cycle progression. In particular, Wnt/β-catenin is involved in the transcription process of cancer-related genes, and the signaling mechanism of Wnt/β-catenin is activated in various carcinomas including gastrointestinal cancer. Therefore, it has been reported that anticancer effects are obtained by inhibition of Wnt/β-catenin signaling when tankyrase is inhibited. In fact, attempts have been made to develop tankyrase inhibitors as anticancer agents.

Accordingly, the compound of Formula I or a pharmaceutically acceptable salt thereof can inhibit PARP1/2 like olaparib, which is used as a conventional standard treatment, and additionally inhibit tankyrase as well. Therefore, it can be seen that it acts by a mechanism different from that of olaparib and the like.

In the present invention, the pharmaceutically acceptable salt of the compound of Formula I is a useful acid addition salt formed from a pharmaceutically acceptable free acid. Acid addition salts are prepared by conventional methods, for example, by dissolving the compound in an excess of an aqueous acid solution and precipitating the salt using a water-miscible organic solvent such as methanol, ethanol, acetone or acetonitrile. That is, it can be prepared by heating equal molar amounts of the compound and an acid or alcohol (eg, glycol monomethyl ether) in water, then evaporating the solvent from the mixture and drying it, or suction filtering the precipitated salt.

At this time, organic acids and inorganic acids may be used as the free acid. Inorganic acid may include hydrochloric acid, phosphoric acid, sulfuric acid, nitric acid and the like. Organic acid may include methanesulfonic acid, p-toluenesulfonic acid, acetic acid, trifluoroacetic acid, maleic acid, succinic acid, oxalic acid, benzoic acid, tartaric acid, fumaric acid, manderic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid and the like, but is not limited thereto.

In particular, the citrate of the compound of Formula I may be preferably used.

In one embodiment of the present invention, as a pharmaceutically acceptable salt of the compound of Formula I, anhydrous, monohydrate, or dihydrate of the citrate of the compound of Formula I may be used, and a crystalline or amorphous form, or a mixed form of crystalline and amorphous form may be used.

Various forms of pharmaceutically acceptable salts of the compound of Formula I can be prepared by methods known in the art.

In one embodiment of the present invention, the PARP inhibitor to which the patient with solid cancer is resistant include olaparib, rucaparib, niraparib, talazoparib and the like, which are anticancer drugs used in standard treatment, but are not limited thereto.

In one embodiment of the present invention, the PARP inhibitor may be olaparib.

In one embodiment of the present invention, the patient with solid cancer may be a patient with a Homologous Recombination Deficiency tumor with BRCA1/2 mutation.

In one embodiment of the present invention, the BRCA1/2 mutation may be germline mutation or somatic mutation.

In one embodiment of the present invention, the patient may be a patient who has a BRCA1/2 mutation, and initially responds to a PARP inhibitor, then acquires resistance during the treatment process and does not respond to the PARP inhibitor, or whose cancer has recurred.

In one embodiment of the present invention, the patient may be a patient show has a BRCA1/2 mutation, but did not respond to the PARP inhibitor.

In one embodiment of the present invention, the patient may be a patient who does not have a BRCA1/2 mutation, and has not previously responded to the PARP inhibitor, or whose cancer has recurred.

In one embodiment of the present invention, the solid cancer may be progressive solid cancer, recurrent solid cancer or metastatic solid cancer.

In one embodiment of the present invention, the solid cancer may be breast cancer, prostate cancer, pancreatic

cancer, ovarian cancer, progressive ovarian cancer, high-grade serous ovarian cancer(including fallopian tubal cancer or primary peritoneal cancer), and breast cancer, prostate cancer, pancreatic cancer that have metastasized from primary cancer, ovarian cancer, but is not limited thereto.

In one embodiment of the present invention, the solid cancer may be ovarian cancer, and metastatic cancer that has spread from primary ovarian cancer.

The pharmaceutical composition according to the present invention may further comprise one or more pharmaceutically acceptable carriers or one or more excipients and/or diluents.

Examples of the pharmaceutically acceptable carriers include, but are not limited to, solids and/or liquids such as ethanol, glycerol, water, and the like. The amount of carrier in the pharmaceutical composition of the present invention may range from about 5% to about 99% by weight based on the total weight of the composition. Types of pharmaceutically acceptable excipients and diluents include non-toxic and compatible fillers, binders, disintegrants, buffers, preservatives, wetting agents, bulking agents, antioxidants, lubricants, flavoring agents, thickeners, colorants, surfactants, emulsifiers, and suspending agents, etc, but is not limited thereto. Such excipients and diluents include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil, but are not limited thereto. It will be apparent to the skilled person that all other pharmaceutically acceptable carriers, excipients and diluents may be used.

A pharmaceutical composition containing the compound or salt thereof of the present invention may be formulated in the form of oral formulations such as tablets, powders, granules, pills, capsules, suspensions, emulsions, internal solutions, emulsions and syrups, external preparations, suppositories or sterile injection solutions according to a conventional method, and used.

The pharmaceutical composition according to the present invention may be in the form of a sterile injectable preparation as a sterile injectable aqueous or oleaginous suspension. This suspension may be formulated according to techniques known in the art using suitable dispersing or wetting agents (e.g., Tween 80) and suspending agents. The sterile injectable preparation may be a sterile injectable solution or suspension in a non-toxic parenterally acceptable diluent or solvent (e.g., a solution in 1,3-butanediol). Acceptable vehicles and solvents include mannitol, water, Ringer's solution, or isotonic sodium chloride solution. In addition, sterile fixed oils may conveniently be employed as a solvent or suspending medium. For this purpose, any bland fixed oil may be employed including synthetic mono- or diglycerides. Fatty acids such as oleic acid and its glyceride derivatives can be usefully employed in injectable preparations as well as pharmaceutically acceptable natural oils (e.g., olive oil or castor oil), especially polyoxyethylated ones thereof.

The pharmaceutical composition according to the present invention may be administered orally in any orally acceptable form including, but not limited to, capsules, tablets, and aqueous suspensions and solutions.

The composition for parenteral administration of the pharmaceutical composition of the present invention may be prepared in the form of a suppository or injection for rectal administration. Suppository compositions can be prepared by mixing the compound of the present invention with a suitable non-irritating excipient that is solid at room temperature but liquid at rectal temperature. Such materials may include, but are not limited to, cocoa butter, beeswax, and polyethylene glycol.

In the case of an injectable composition, the compound of the present invention may be included as an active ingredient in a conventional excipient for injection, and the route of administration may be intravenous injection, intramuscular injection, subcutaneous injection, etc., but is not limited thereto.

The novel compound described above in the pharmaceutical composition of the present invention is contained in a therapeutically effective amount or a prophylactically effective amount. A preferred dosage of the compound according to the present invention varies depending on the condition and weight of the patient, the severity of the disease, the type of drug, the route and duration of administration, but can be appropriately selected by those skilled in the art. However, for desirable effects, the compound of Formula I of the present invention or a pharmaceutically acceptable salt thereof is administered in an amount of 0.0001 to 1000 mg, 0.01 to 500 mg, 0.1 to 300 mg, 1 to 200 mg, or 50 to 200 mg per day. It can be administered once or divided into several times. In the composition of the present invention, the compound of Formula I may be formulated in an amount of 0.0001 to 50% by weight based on the total weight of the composition.

The pharmaceutical composition of the present invention may further contain at least one active ingredient exhibiting the same or similar medicinal effect in addition to the compound represented by Formula I, its optical isomer, its racemate or its pharmaceutically acceptable salt.

In addition, the present invention provides the use of the compound of Formula I or a pharmaceutically acceptable salt thereof for the preparation of a drug for preventing or treating solid cancer resistant to PARP inhibitors.

The compound represented by Formula I or a pharmaceutically acceptable salt thereof for the preparation of pharmaceuticals can be mixed with pharmaceutically acceptable adjuvants, diluents, carriers, etc., and is prepared as a complex preparation with other active agents to have a synergistic effect.

In addition, the present invention provides a method for preventing or treating solid cancer resistant to PARP inhibitors by administering an effective amount of the compound of Formula I or a pharmaceutically acceptable salt

thereof to mammals including humans.

The prophylactic or therapeutic method of the present invention includes not only treating the disease itself before the onset of symptoms, but also inhibiting or avoiding its symptoms, by administering the compound represented by Formula I or a pharmaceutically acceptable salt thereof. In the management of disease, the prophylactic or therapeutic dose of a particular active ingredient will vary depending on the nature and severity of the disease or condition and the route by which the active ingredient is administered. Dosage and frequency of administration will vary according to the age, weight and response of the individual patient. A suitable dosage regimen can be readily selected by those skilled in the art who take these factors into account. In addition, the preventive or therapeutic method of the present invention may further include the administration of a therapeutically effective amount of an additional active agent useful for disease treatment together with the compound represented by Formula I. The additional active agent may exhibit a synergistic or additive effect with the compound of Formula I or a pharmaceutically acceptable salt thereof.

Matters mentioned in the pharmaceutical composition, use, and treatment method of the present invention are equally applied unless they contradict each other.

The pharmaceutical composition of the present invention may be provided in the form of a kit including instructions and the like.

Unless otherwise indicated, all numbers used in the specification and claims, whether stated or not, are to be understood in all instances as being modified by the term "about." Also, the precise numerical values used in the specification and claims are to be understood as forming additional embodiments of the present disclosure. Efforts have been made to ensure the accuracy of the figures disclosed in the examples. However, any measured number inherently may contain certain error values resulting from the standard deviation found in its respective measurement technique.

Hereinafter, the present invention will be described in more detail through examples. These examples are only for explaining the present invention in more detail, and it will be apparent to those skilled in the art that the scope of the present invention is not limited by these examples according to the gist of the present invention.

In the present invention, the citrate of the compound of Formula I can be prepared by methods known in this field, a method disclosed in Korean Patent Application No. 10-2021-0064416 or a method disclosed in an application filed on the same date as the present invention as an application claiming priority based on the above application. For example, the method for preparing the citrate of the compound of Formula I is as follows.

Preparative Example 1:

Preparation of 6-{4-[(5-oxo-1,2,3,4,5,6-hexahydrobenzo[h][1,6]naphthyridin-8-yl)methyl]piperazin-1-yl}nicotinonitrile citrate(citrate of compound of Formula I)

Methanol(25.7L) and purified water(25.7L) were added to 6-{4-[(5-oxo-1,2,3,4,5,6-hexahydrobenzo[h][1,6]naphthyridin-8-yl)methyl]piperazin-1-yl}nicotinonitrile(Compound of Formula I, 7.34kg, 18.32mol). Citric acid (5.28 kg, 27.49 mol) was dissolved in a 1:1 mixed solution (22 L) of methanol and purified water and then added thereto. After stirring for 30 minutes at 15 to 25°C, the temperature was raised to 60°C, and stirred at 60 to 70°C for 2 hours. After cooling to room temperature, the mixture was filtered to obtain citrate monohydrate of the compound of Formula I (10.7kg, 95.8%).

Ethanol(2.5L), acetone(2.5L) and isopropanol(2.5L) were added to the citrate monohydrate of the compound of Formula I(500g,0.82mol), and then purified water(20mL) was added thereto. After raising the temperature to 55°C, it was stirred for 4 hours at 55 to 75°C. After cooling to 25°C or lower, the mixture was stirred for 30 minutes. The resulting solid was filtered to obtain citrate anhydride of the compound of Formula I (470 g, yield 96.7%).

**Example** 1: Analysis of anticancer effect of compound of Formula I (in vitro experiment)

In order to analyze the anticancer effect of citrate of 6-{4-[(5-oxo-1,2,3,4,5,6-hexahydrobenzo[h][1,6]naphthyridin-8-yl)methyl]piperazin-1-yl}nicotinonitrile) of the compound of Formula I in homologous recombination deficient tumors, cell division analysis using various cell lines was performed.

In this assay, HCC1937, SNU-251, BT474 and SNU-119 cell lines were used as BRCA mutation-positive ovarian cancer or breast cancer cell lines, and as primary cells of BRCA mutation-positive ovarian cancer, CHA- OVA-13 cells isolated from actual BRCA1 mutation-positive ovarian cancer patients (ovarian cancer primary cells showing acquired resistance to olaparib) were used.

First, whether each cell was a cell line resistant to olaparib is checked by measuring $IC_{50}$. At this time, cell lines with an $IC_{50}$ of 50uM or more for olaparib are selected as PARP inhibitor-resistant cell lines suitable for this study.

The following experiments were performed using various PARP inhibitors on the olaparib-resistant cell line. Specifically, each cell was suspended in a culture medium, dispensed into a 96 well plate, and cultured for 24 hours at 5% $CO_2$ and 37°C. Then, olaparib, niraparib, talazoparib and the citrate of the compound of Formula I were treated in

a dose dependent manner, and MTT reagent was added after 72 hours, and stop buffer (10% SDS) was added after 3 hours. After reaction for 2 to 4 hours, the absorbance was measured at 595 nm, and the $IC_{50}$ value was calculated at the concentration at which each drug inhibited cell growth by 50%.

At the same time, in order to confirm the apoptosis mechanism and anticancer activity mechanism of cells treated with each drug at the molecular level, the amount of proteins related to apoptosis, such as pATR, pCHK1, pAKT, tankyrase, cleaved caspase 3, and cleaved PARP protein, were analyzed by immunoblot method.

**Example 2: Analysis of anticancer effect(in vitro experiment)**

In order to analyze the anticancer effect of citrate of (6-{4-[(5-oxo-1,2,3,4,5,6-hexahydrobenzo[h][1,6]naphthyridin-8-yl)methyl]piperazin-1-yl}nicotinonitrile of the compound of Formula I on BRCA Wild type and Mutation type cells, cell division analysis using various cell lines was performed.

**(1) Experimental method**

In this assay, wild-type BRCA ovarian cancer cell lines OVCAR-3, OVCAR-5, SKOV3, NCI/ADR-RES, A2780, A2780 CR (carboplatin-resistant-inducing cell line) and OVCA433R (olaparib-resistant cell line) and mutation type BRCA1 ovarian cancer cell line, SNU-251, were used. After culturing the above cell line in a culture medium (RPMI-1640 + 10% heat-inactivated FBS + 1% antibiotic-antimycotic) under the condition of 37°C 5% $CO_2$, it was removed from the cell culture dish and cultured overnight in a 6-well plate to attach the cells. The citrate of the compound of Formula I, olaparib, and niraparib were sequentially diluted from high concentrations and treated at various concentrations, and the remaining empty wells were treated with vehicle control. On the 14th day, crystal violet was treated to stain live cell colonies. After washing with PBS and sufficiently drying at room temperature, the number of stained colonies was counted and recorded using a microscope and the naked eye. The $IC_{50}$ value was calculated based on the concentration capable of inhibiting colony formation by 50%($IC_{50}$: an inhibitory concentration to achieve 50% colony formation inhibition) by converting the relative number of colonies in the experimental group into % when the vehicle control was assumed to be 100%.

**(2) Experimental result**

The experimental results are shown in Table 1 below and FIG. 1.

[Table 1]

| $IC_{50}$([con.], nM) | | | |
|---|---|---|---|
| Cell strain/Drug | Citrate of compound of Formula I | Olaparib | Niraparib |
| SNU-251 (mBRCA1) | 0.8526 ± 0.19 | 465.55 ± 35 | 1147.6 ± 279.17 |
| OVCAR3 | 2.0845 ± 0.01 | 2.39 ± 0.09 | 72.77 ± 11.19 |
| A2780 | 3.6 ± 0.21 | 219.15 ± 10.82 | 158.9 ± 10.61 |
| A2780 CR (Chemo R) | 7.4 ± 1.28 | 540.9 ± 32.81 | 306.65 ± 69.79 |
| SKOV3 | 11.92 ± 3.59 | 1463.5 ± 120.92 | 1121 ± 16.97 |
| NCI/ADR-RES (Drug R) | 25.68 ± 6.02 | 756.95 ± 347.97 | 456.95 ± 81.95 |
| OVCAR5 | 299.55 ± 117.45 | 1832 ± 12.73 | 1310.1 ± 681.51 |
| OVCA433R (Ola-R) | 11321 | 53717 | ND |

As shown in Table 1 and the graph of FIG. 1, it can be seen that the citrate of the compound of Formula I inhibits the growth of cancer cells even at a significantly lower concentration than olaparib or niraparib, regardless of BRCA mutation. In particular, in the NCI/ADR-RES cell line showing drug resistance due to overexpression of the drug efflux pump or the A2780-CR cell line inducing carboplatin resistance, cancer growth was effectively inhibited at significantly lower concentrations than other PARP inhibitors.

**Example 3: Evaluation of efflux ratio by P-gp of citrate of compound of Formula I**

P-glycoprotein (P-gp) is one of the drug transporters that determine the absorption and efflux of various drugs. These processes of absorption and efflux of drugs affect the concentration of the drug in plasma and tissues and ultimately

the final effect of the drug.

Compounds with high P-gp substrate specificity are known to cause reduced drug accumulation in multidrug-resistant cells and often mediate the development of resistance to anticancer drugs. PARP inhibitors such as olaparib, rucaparib, niraparib, and talazoparib are all known compounds with high P-gp substrate specificity.

In this experiment, the inventors compared and evaluated the efflux ratio of the citrate of the compound of Formula I in P-gp and that of olaparib, a representative PARP inhibitor, in order to identify the mechanism by which the citrate of the compound of Formula I exhibits excellent effects in the treatment of PARP inhibitor-resistant cancer.

**(1) Experimental method**

A permeability study was conducted to measure the efflux ratio of the citrate of the compound of Formula I and olaparib (AZD-2281). 200 μL of a culture medium in which the number of $CaCO_2$ cells is $5 \times 10^4$/well per each insert was dispensed on the apical side of a transwell insert having a diameter of 6.5 mm in a 24-well plate. 800 μL of the culture solution was placed on the basolateral side of the well plate so that the lower part of the insert was submerged. After 21 days of culture, the culture medium of the insert and the plate was removed, the citrate of the compound of Formula I was diluted in the culture medium at concentrations of 1 μM, 10 μM, and 50 μM, respectively, and olaparib (AZD-2281) was diluted in the culture medium at a concentration of 10 μM. 250 μL of each dilution was applied to the top of the transwell insert, and 800 μL of drug-free culture was added to the bottom (Papp A→B measurement). To examine the effect of the P-gp pump, 800 μL of culture medium containing the same drug concentration was applied to the base, and culture medium without drug was placed on the upper layer of the insert (Papp B→A measurement). Samples of 100 μL taken from each supernatant or basolateral portion were analyzed at the specified times, 0, 30, 60, 120, and 180 minutes.

**(2) Experimental result**

The experimental results are shown in Table 2 below.

[Table 2]

| Drug | | Papp A→B (X$10^6$ cm/s) | Papp B→A ($\times 10^6$ cm/s) | Efflux ratio (B→A / A→B) |
|---|---|---|---|---|
| Olaparib (10 μM) | | 1.7 | 44.8 | 26.35 |
| citrate of compound of Formula I | 1 μM | 19.8 | 44.1 | 2.23 |
| | 10 μM | 18.1 | 49.9 | 2.76 |
| | 50 μM | 39.7 | 41.2 | 1.04 |
| Note) A: Cell apical side, B: Cell basolateral side | | | | |

As confirmed in Table 2, the efflux ratio of the citrate of the compound of Formula I was found to be about 1/10 of that of olaparib, a PARP inhibitor of the P-gp substrate.

**(3) Evaluation of experimental result**

From the above experimental results, it can be seen that the citrate of the compound of Formula I of the present invention is not a P-glycoprotein (P-gp) substrate.

With this mechanism of action, the citrate of the compound of Formula I seems to be able to overcome multi-drug resistance and show better anticancer effects, unlike PARP inhibitors such as olaparib, rucaparib, niraparib, and talazoparib, which are known as P-gp substrates.

Specifically, in Example 2, the citrate of the compound of Formula I inhibited the growth of cancer cells in various types of wild-type BRCA ovarian cancer cell lines and mutation-type BRCA ovarian cancer cell lines even at concentrations significantly lower than those of olaparib or niraparib. It seems that the action mechanism by the efflux ratio by P-gp also contributed to this excellent effect in part. In particular, according to the results of Example 2, the citrate of the compound of Formula I in the NCI/ADR-RES cell line, which exhibits drug resistance due to overexpression of the drug efflux pump, effectively inhibits cancer growth at significantly lower concentrations than other PARP inhibitors. Therefore, these experimental results are judged to more clearly explain that the non-P-gp substrate of the compound of Formula I contributes in part to the excellent anticancer effect.

Furthermore, PARP inhibitors are known to have a high ratio of congenital or acquired resistance acquisition, and as a mechanism explaining this cause, a mechanism of increasing drug efflux by increasing ABC receptors (ABC transporter) is known.

Therefore, the mechanism of action related to the efflux rate of the citrate of the compound of Formula I is considered to theoretically support the fact that the compound of Formula I can be effectively used in the treatment of cancers resistant to other PARP inhibitors such as olaparib, rucaparib, niraparib, and talazoparib. And, this logic is clearly supported by the experimental results of Example 4 (analysis of anti-cancer effect-Xenograft model), Example 5 (analysis of anti-cancer effect-Xenograft model), and Example 6 (phase I clinical trial-NOV140201) below.

In conclusion, the citrate of the compound of Formula I of the present invention provides an excellent anticancer effect with a mechanism of action different from the efflux mechanism of drugs such as olaparib, rucaparib, niraparib, and talazoparib, which are other PARP inhibitors, and provides excellent effects on cancer resistant to these PARP inhibitors.

**Example 4: Efficacy evaluation by inhibition of Wnt signaling activity related to olaparib resistance**

**(1)Experimental method**

TOP/FOP-flash luciferase reporter assay was performed to measure the activation of Wnt signaling in the ovarian cancer cell line PEO1 and the ovarian cancer cell line PEO1-OR that acquired olaparib resistance. As for the olaparib-resistant-acquired cell line, the PEO1 cell line was treated sequentially from a low olaparib concentration (10 nM) to a high concentration of 8 $\mu$M, and the surviving cell line is referred to as PEO1-OR. TOP/FOP-flash luciferase reporter assay uses the principle that $\beta$-catenin, which is produced when Wnt is activated, moves into the nucleus and binds to the TCF/LEF promoter site to transcribe genes affected by Wnt. This gene is then replaced with luciferase, and is to measure the degree of Wnt activation by measuring the luminescence converted by a non-luminescent substrate by luciferase. TOP-flash is an experimental plasmid in which transcription of luciferase occurs because $\beta$-catenin can bind to the promoter site of the TCF promoter. FOP-flash is used as a transfection control plasmid to measure the basal level of fluorescence because $\beta$-catenin cannot bind to the promoter site by introducing a mutation in the TCF promoter.

TOP-flash or FOP-flash plasmids were transfected into PEO1, an ovarian cancer cell line, and PEO1-OR cell line, an ovarian cancer cell line that acquired olaparib resistance. The transfected PEO1 and PEO1-OR cell lines were exposed to vehicle control and the citrate of the compound of Formula I at 400 nM, 10 $\mu$M, and 50 $\mu$M, respectively, for 72 hours of transfection, then the cells were lysed and luciferase substate was added. The degree of luminescence coming off the substrate was measured and recorded using a fluorescence reader.

**(2) Experimental result**

The experimental results are shown in FIG. 2.

As shown in the left graph of FIG. 2, as a result of relative comparison of the strength of TOP signaling between the ovarian cancer cell line PEO1 and the ovarian cancer cell line PEO1-OR that has acquired olaparib resistance, the cell line of PEO1-OR that has acquired olaparib resistance showed a 5-fold higher TOP signal transduction intensity compared to other cell lines. These results indicate that Wnt signaling was activated by acquisition of olaparib resistance in the PEO1-OR cell line.

In addition, it can be confirmed that Wnt signaling is reduced in proportion to the concentration of the citrate of the compound of Formula I compared to the control group when the PEO1-OR cell line in which Wnt is activated was treated with the citrate of the compound of Formula I from the right graph of FIG. 2.

In conclusion, from the above experiment, when the PEO1 cell line acquires olaparib resistance, Wnt signaling increases (see the graph on the left of FIG. 2), and this increased signaling decreases in proportion to the dose due to the tankylase inhibitory ability of the citrate of the compound of Formula I (see the graph on the right of FIG. 2).

**(3) Evaluation of experimental result**

Recently, studies have been reported that the resistance mechanism of PARP inhibitors is related to the Wnt signaling pathway. In other words, it is known that the Wnt signaling pathway is activated as a mechanism of resistance development by PARP inhibitors. Therefore, resistance to PARP inhibitors can be overcome by selecting anticancer drugs that can block the Wnt signaling pathway in cancer cells that have acquired resistance to PARP inhibitors.

Unlike existing PARP inhibitors such as olaparib, the citrate of the compound of Formula I of the present invention has dual inhibition of PARP and Tankyrase (TNK). That is, existing PARP inhibitors are known to activate the Wnt signaling pathway in the development of resistance, whereas the citrate of the compound of Formula I of the present invention effectively inhibits Wnt signaling by TNK inhibitory action.

Therefore, it can be seen from this fact that the citrate of the compound of Formula I of the present invention can be effectively used for the treatment of cancers that have acquired resistance to PARP inhibitors.

**Example 5: Analysis of anticancer effect(Xenograft model)**

In order to prove that the citrate of the compound of Formula I can be used for the treatment of solid cancer that is actually resistant to PARP inhibitors, a xenograft model using cells derived from BRCA mutation-positive ovarian cancer was created and the effects of PARP inhibitor (olaparib) and the citrate of the compound of Formula I were compared.

**(1) Experimental method**

PDX-GFTP 1016 is a cell derived from the primary tissue of a patient with stage IIIC high-grade serous ovarian cancer and has TP53 and BRCA2 mutations. To facilitate the tracking of this cancer cell, green fluorescent protein/luciferase was expressed (PDX-1016 GTFP 1016 GFP/luc) and surgically injected into the right ovary between the intrabursals, and then the colonization of cancer cells was monitored for 4 weeks before drug treatment. After 4 weeks, regrowth of cancer cells in mice treated with 50 mg/Kg of olaparib for 28 days was defined as olaparib-resistant PDX-1016 GTFP 1016 GFP/luc (Ola-R-GTFP-1016).

Cells of Ola-R-GTFP-1016, an olaparib-resistant PDX established by the above method, were surgically transferred to above the right ovarian bursa at the same site as the original cancer at a concentration of $1 \times 10^6$ cells/ml, as schematically shown in FIG. 3A, and stabilized for 4 weeks.

After the above 4 weeks of stabilization, based on the flux (photons per second) collected image data such as light intensity of GFP/Luciferase expressed by cancer through in vivo flux imaging (IVIS spectrum in vivo imaging system, PerkinElmer), cancer distribution and cancer growth were observed.

At this time, using in vivo flux imaging, transplanted mice with similar growth rates of cancer were randomly divided into a control group (vehicle treatment group) and a group treated with 25mg/kg of the citrate of the compound of Formula I, and oral administration was performed once a day. Changes in flux were recorded every week, and after 4 weeks, mice were sacrificed, and anticancer activity was measured by measuring the volume of ascites, the volume of cells in the ascites, and the number of cancer metastases to the lymph.

**(2) Experimental result**

The experimental results are shown in FIG.3 (B to E).

In FIG. 3B, the fact that the fluorescence of the abdomen is shown as a strong spectrum color indicates that cancer proliferation is actively occurring in the ascites. That is, FIG. 3B shows that cancer growth is effectively suppressed in the group treated with the citrate of the compound of Formula I compared to the control group (vehicle treatment group).

In the graph of FIG. 3C, the in vivo fluorescence recorded from 2 weeks after drug treatment was used as a standard for basal level, and the change in relative fluorescence was observed through in vivo imaging in the abdomen of transplanted mice until 4 weeks of drug treatment.

It can be seen from graph of FIG. 3C that the growth of cancer cells in the group treated with the citrate of the compound of Formula I was significantly inhibited compared to the control group (vehicle treatment group).

From the data supporting that treatment with the citrate of the compound of Formula I inhibits cancer growth more effectively than the control group, the number of cancer nodules and the number of multiple cells were counted to determine the metastatic potential of cancer.

As shown in FIG. 3D, the nodule formation ability of cancer was significantly reduced in the citrate of the group treated with the compound of Formula I treatment group compared to the control group. In addition, as shown in of FIG. 3E, the total number of cells generated in ascites was significantly decreased in the group treated with the citrate of the compound of Formula I than in the control group.

From these results, it can be confirmed that the group treated with the citrate of the compound of Formula I inhibited the formation and metastasis of cancer more effectively than the control group.

**Example 6: Analysis of anticancer effect(xenograft model)**

**(1) Experimental method**

CHA-OVA-13 is a primary cell established from ascites cells of ovarian cancer patients with acquired resistance to olaparib and has a BRCA1 mutation. This CHA-OVA-13 was injected into NOD/SCID mice to grow the size of the original cancer, and when it grew to a certain size, the cancer was removed, cut into small pieces, and then the tumor

was transplanted into the subcutaneous layer of nude mice to establish a mouse model. When the tumor size reached 80 mm3, mice with similar tumor sizes were selected and randomly divided into an olaparib-treated group and the citrate of the compound of Formula I-treated group.

Subsequently, 50 mg/kg of olaparib was orally administered twice a day to the olaparib-treated group, and 50 mg/kg of the citrate of the compound of Formula I was orally administered once a day to the citrate of the compound of Formula I-treated group. At two-day intervals, the size of the tumor was measured the width and height through the caliber and re $Volume = (4/3)\pi \times \left(\frac{Length}{2}\right)^2 \times \left(\frac{Depth}{2}\right)$ calculated by the following equation.

The tumor size of the olaparib-treated group and the citrate of the compound of Formula I-treated group was observed for 18 days. The olaparib-treated group was randomly divided into two groups after drug treatment for 10 days, and one group continued to receive olaparib in the same manner for 8 days (olaparib-treated group), and the other group changed the drug to the citrate of the compound of Formula I and administered orally once a day at 50 mg/kg for 8 days (olaparib-the citrate of the compound of Formula I replacement group) to observe the effect on tumor size.

**(2) Experimental result**

The experimental results are shown in FIG. 4. As shown in the graph of FIG. 4B, it can be confirmed that the tumor growth in the citrate of the compound of Formula I-treated group was noticeably slower than that of the olaparib-treated group in observation for a total of 18 days.

In addition, in the case of the olaparib-the citrate of the compound of Formula I replacement group, which was treated by replacing the olaparib with the citrate of the compound of Formula I at 10 days after dividing the olaparib treatment group into two, the tumor growth rate was gradually slowed compared to the single olaparib treatment group, and from around the 13th day, the tumor growth inhibitory effect similar to that of the citrate of the compound of Formula I single treatment group was shown.

These results indicate that continued treatment of olaparib to primary cell xenografts of olaparib-resistant patients did not inhibit tumor growth, but treatment with the citrate of the compound of Formula I significantly slowed down tumor growth.

From these results, it can be confirmed that the citrate of the compound of Formula I provides an excellent effect on inhibiting the growth of olaparib-resistant cancer tissue.

**Example 7: Phase I clinical trial**

Phase I clinical trial was conducted to evaluate the stability, tolerability, pharmacokinetic and pharmacodynamic properties and efficacy of the citrate of (6-{4-[(5-oxo-1,2,3,4,5,6-hexahydrobenzo[h][1,6]naphthyridin-8-yl)methyl]piperazin-1-yl}nicotinonitrile) (6-{4-[(5-oxo-1,2,3,4,5,6-hexahydrobenzo[h][1,6]naphthyridin-8-yl)methyl]piperazin-1-yl}nicotinonitrile of the compound of Formula I. The patient group consists of patients aged 19 years or older with histologically or cytologically confirmed progressive solid cancer who is refractory to standard treatment or cannot receive standard treatment. Patients whose expected survival period is 12 weeks or longer and whose proper hematological and liver function has been confirmed through such as general blood tests were selected.

Selected patients provided written consent according to institutional and FDA regulations. 22 patients were enrolled in the dose escalation cohort and 40 patients were enrolled in the dose expansion cohort(citrate of 6-{4-[(5-oxo-1,2,3,4,5,6-hexahydrobenzo[h][1,6]naphthyridin-8-yl)methyl]piperazin-1-yl}nicotinonitrile). The dose expansion cohort received 50 mg/day, 100 mg/day, 150 mg/day, and 200 mg/day.

Patients in all cohorts were evaluated for safety evaluation, blood collection at regular intervals, biomarker analysis through biopsy (tumor specimens), and genetic analysis (blood PBMC and tumor specimens) and tumor response (every 6 weeks) according to regulations. In addition, follow-up was conducted according to institutional and Ministry of Food and Drug Safety regulations even after administration was completed.

Among the patients enrolled in the dose expansion cohort (150mg/day), a 64-year-old female patient was initially diagnosed with metastatic ovarian cancer (stage 3) and had extensive solid cancer tissues including ovaries surgically resected, and is a homologous recombination deficiency tumor patient with germline BRCA1 mutation.

The patient showed little response to various anticancer drugs such as gemcitabine, cisplatin, and paclitaxel, and in particular, was judged to have resistance to olaparib as an increase in the size of the remaining cancer tissue (target lesion) and a new lesion (progressive disease) were observed immediately after olaparib administration. Then, neoplatin, a cisplatin-type drug, was administered for 2 months, but the patient did not respond. Afterwards, the patient registered for this clinical trial and started taking the citrate of the compound of Formula I alone at 150 mg/day, and it was confirmed that the size of the lesion (solid cancer metastasized to the liver) decreased by more than 30% compared

to the baseline by periodic CT scans while taking the drug.

**Example 8: Phase II clinical tiral**

Phase II clinical trial is conducted to evaluate whether the citrate of (6-{4-[(5-oxo-1,2,3,4,5,6-hexahydrobenzo[h][1,6]naphthyridin-8-yl)methyl]piperazin-1-yl}nicotinonitrile) of the compound of Formula I can be used for the treatment of patients resistant to PARP inhibitors. The patient group consists of patients aged 19 years or older with histologically or cytologically confirmed solid cancer who have been confirmed to be HRD (homologous recombination deficiency) positive. Specifically, the patients were patients with high-grade (Grade 2 or 3) serous epithelial ovarian cancer, fallopian tubal cancer or primary peritoneal cancer, whose cancer has recurred or progressed after receiving more than 2 lines of anticancer treatment for their tumor before participating in this clinical trial.

The anticancer treatment specifically refers to treatment with one or a combination of gemcitabine, doxorubicin, topotecan, carboplatin, oxaliplatin, cisplatin, bevacizumab, or a PARP inhibitor.

As a subject, patients whose expected survival period is 12 weeks or longer and whose proper hematological function, renal function, and liver function has been confirmed through such as general blood tests are selected.

Selected patients provide written consent according to institutional and FDA regulations. The patient includes those who show sensitivity to platinum-based therapeutics in previous treatment history, or those who have shown sensitivity to platinum-based therapeutics but have failed treatment with existing PARP inhibitors such as olaparib and niraparib. About 60 patients are enrolled, but it is flexible.

The citrate of the compound of Formula I is administered at 100 mg/day, and the dosage can be adjusted if necessary.

Patients in all cohorts are evaluated for safety evaluation, blood collection at regular intervals, genetic analysis (blood PBMC and tumor specimens) and tumor response (every 8 weeks) according to regulations. In addition, follow-up is conducted according to institutional and Ministry of Food and Drug Safety regulations even after administration was completed.

Efficacy, safety, tolerability, PK, etc. can be measured and evaluated by this Phase II clinical trial.

The efficacy includes therapeutic effect of ’the citrate of Formula I of the present invention’ on ’HRD mutation-positive tumor patients who have failed treatment with existing PARP inhibitors, etc.’. More specifically, this Phase II clinical trial will effectively reduce the size of solid cancer in patients who have failed treatment with existing PARP inhibitors.

## Claims

1. A pharmaceutical composition comprising 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for treating or preventing cancer in a patient with solid cancer resistant to a PARP inhibitor.

2. The pharmaceutical composition according to claim 1, wherein the patient with solid cancer has a Homologous Recombination Deficiency(HRD)tumor.

3. The pharmaceutical composition according to claim 2, wherein the patient with solid cancer has BRCA1/2 mutation.

4. The pharmaceutical composition according to claim 3, wherein the BRCA1/2 mutation is germline mutation.

5. The pharmaceutical composition according to claim 3, wherein the BRCA1/2 mutation is somatic mutation.

6. The pharmaceutical composition according to claim 1, wherein the patient with solid cancer does not have BRCA1/2 mutation.

7. The pharmaceutical composition according to claim 1, wherein the PARP inhibitor is at least one selected from olaparib, rucaparib, niraparib, and talazoparib.

8. The pharmaceutical composition according to claim 7, wherein the PARP inhibitor is olaparib.

9. The pharmaceutical composition according to claim 1, wherein the solid cancer is at least one selected from breast cancer, prostate cancer, pancreatic cancer, ovarian cancer, progressive ovarian cancer, high-grade serous ovarian cancer (including fallopian tubal cancer or primary peritoneal cancer), and metastatic cancer that has spread from

primary ovarian cancer,

**10.** The pharmaceutical composition according to claim 9, wherein the solid cancer is ovarian cancer.

**11.** The pharmaceutical composition according to claim 9, wherein the solid cancer is metastatic cancer that has spread from primary ovarian cancer.

**12.** The pharmaceutical composition according to any one of claims 1 to 11, wherein the pharmaceutically acceptable salt of the 6-{4-[(5-oxo-1,2,3,4,5,6-hexahydrobenzo[h][1,6]naphthyridin-8-yl)methyl]piperazin-1-yl}nicotinonitrile is citrate.

**13.** The pharmaceutical composition according to any one of claim 1 to claim 11, wherein the pharmaceutical composition further comprises a pharmaceutically acceptable carrier or excipient.

**14.** 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for the treatment of a patient with solid cancer resistant to a PARP inhibitor.

**15.** A method for treating a patient with solid cancer resistant to a PARP inhibitor by administering an effective amount of 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof.

**16.** An use of 6-{4-[(5-oxo-1,2,3,4,5,6-hexahydrobenzo[h][1,6]naphthyridin-8-yl)methyl]piperazin-1-yl}nicotinonitrile or a pharmaceutically acceptable salt thereof for the manufacture of a drug for the treatment of a patient with solid cancer resistant to a PARP inhibitor.

【Figure 1】

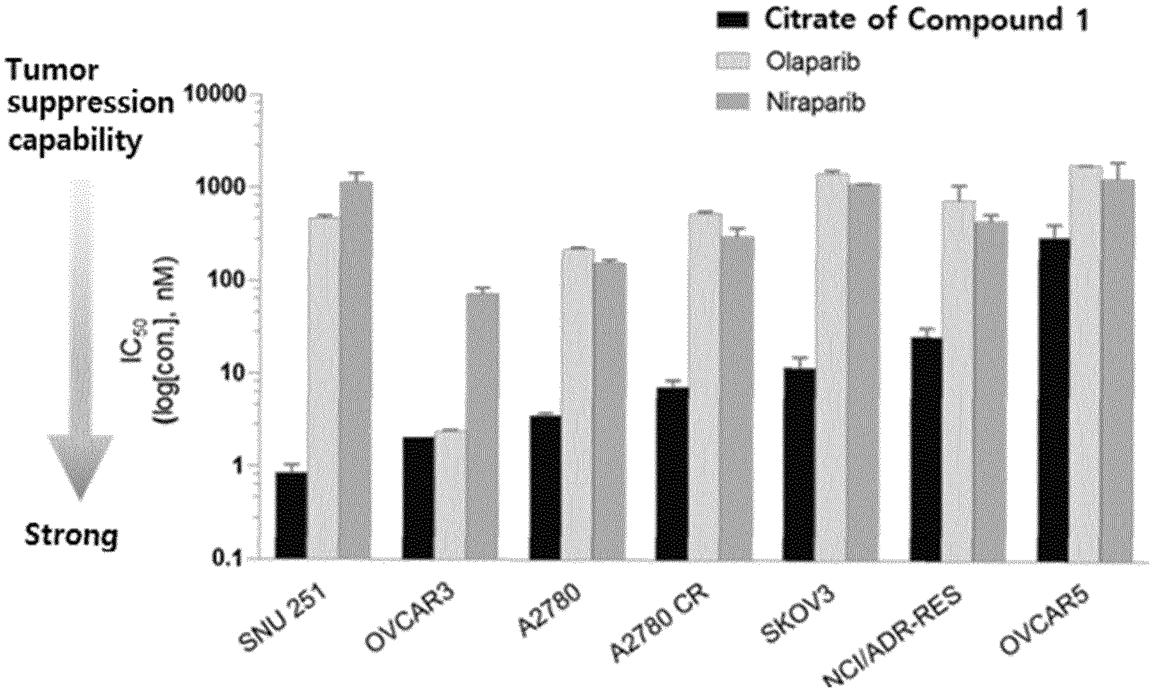


【Figure 2】

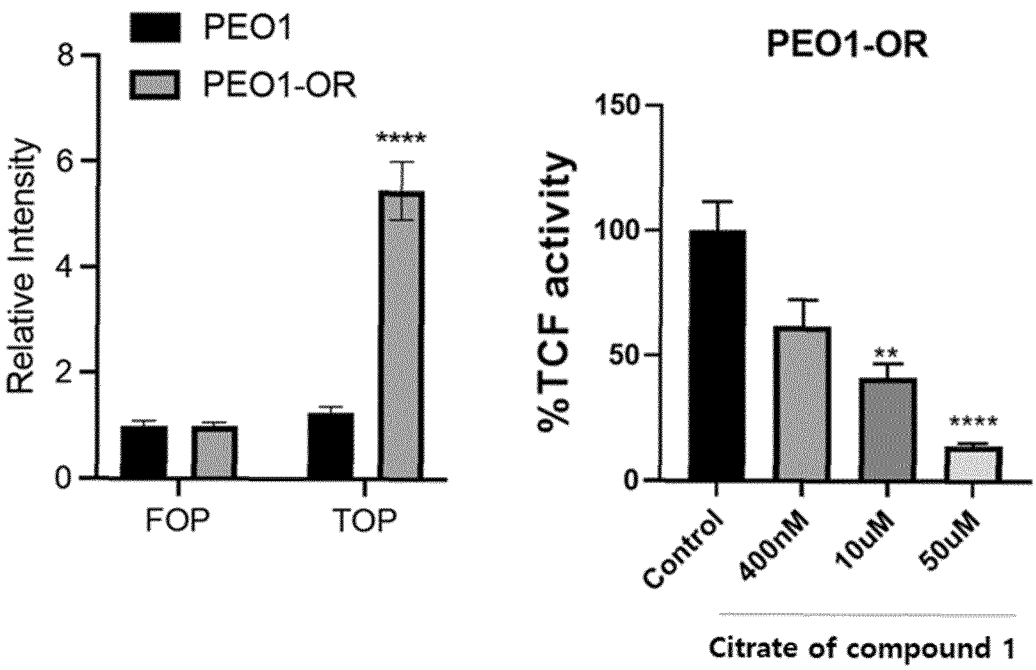

【Figure 3】

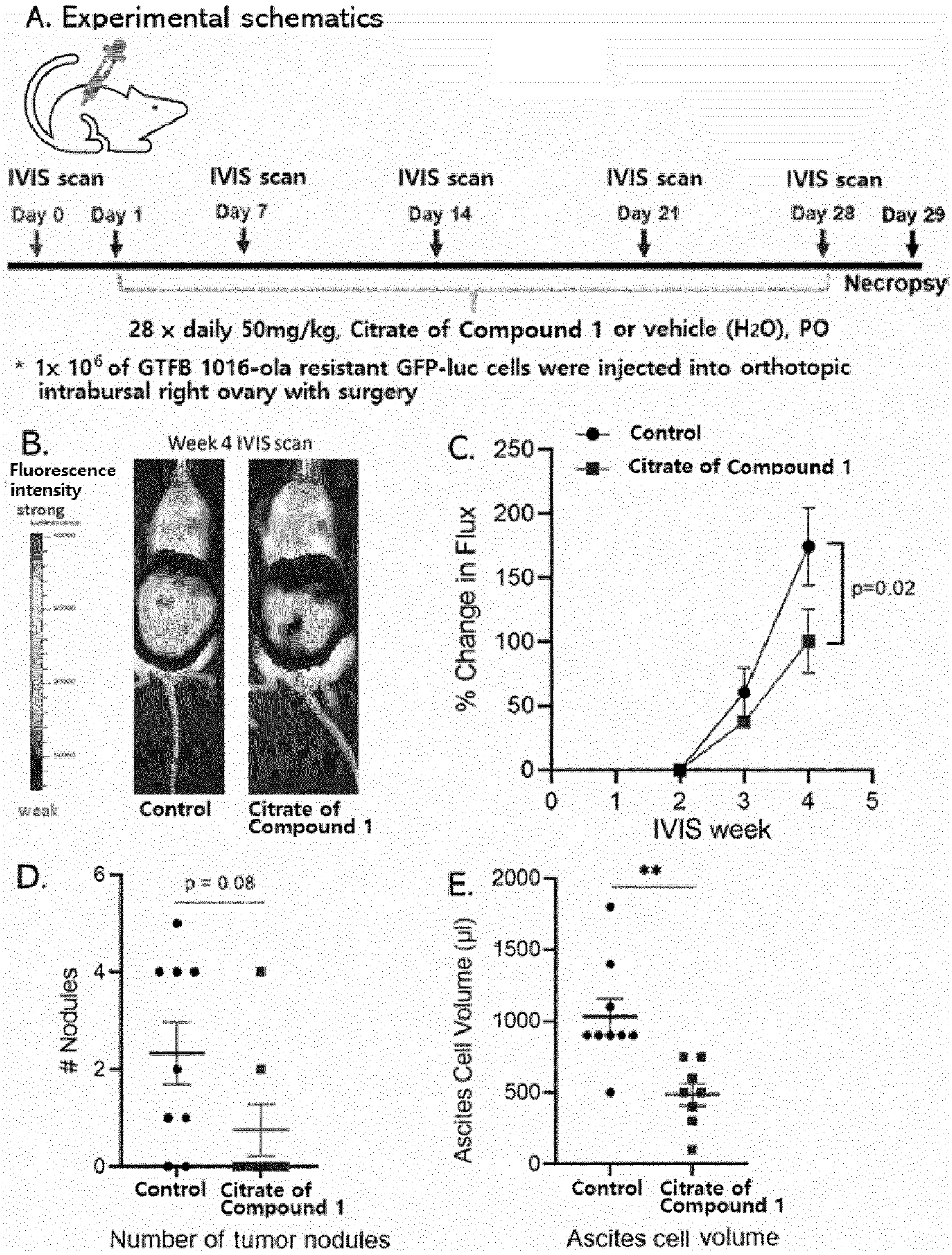
A. Experimental schematics
IVIS scan
IVIS scan
IVIS scan
IVIS scan
IVIS scan
Day 0
Day 1
Day 7
Day 14
Day 21
Day 28
Day 29
Necropsy
28 x daily 50mg/kg, Citrate of Compound 1 or vehicle (H2O), PO
* 1x 10^6 of GTFB 1016-ola resistant GFP-luc cells were injected into orthotopic intrabursal right ovary with surgery
B.
Week 4 IVIS scan
Fluorescence intensity
strong
weak
Control
Citrate of Compound 1
C.
Control
Citrate of Compound 1
% Change in Flux
250
200
150
100
50
0
p=0.02
0 1 2 3 4 5
IVIS week
D.
p = 0.08
# Nodules
6
4
2
0
Control
Citrate of Compound 1
Number of tumor nodules
E.
**
Ascites Cell Volume (μl)
2000
1500
1000
500
0
Control
Citrate of Compound 1
Ascites cell volume

【Figure 4】

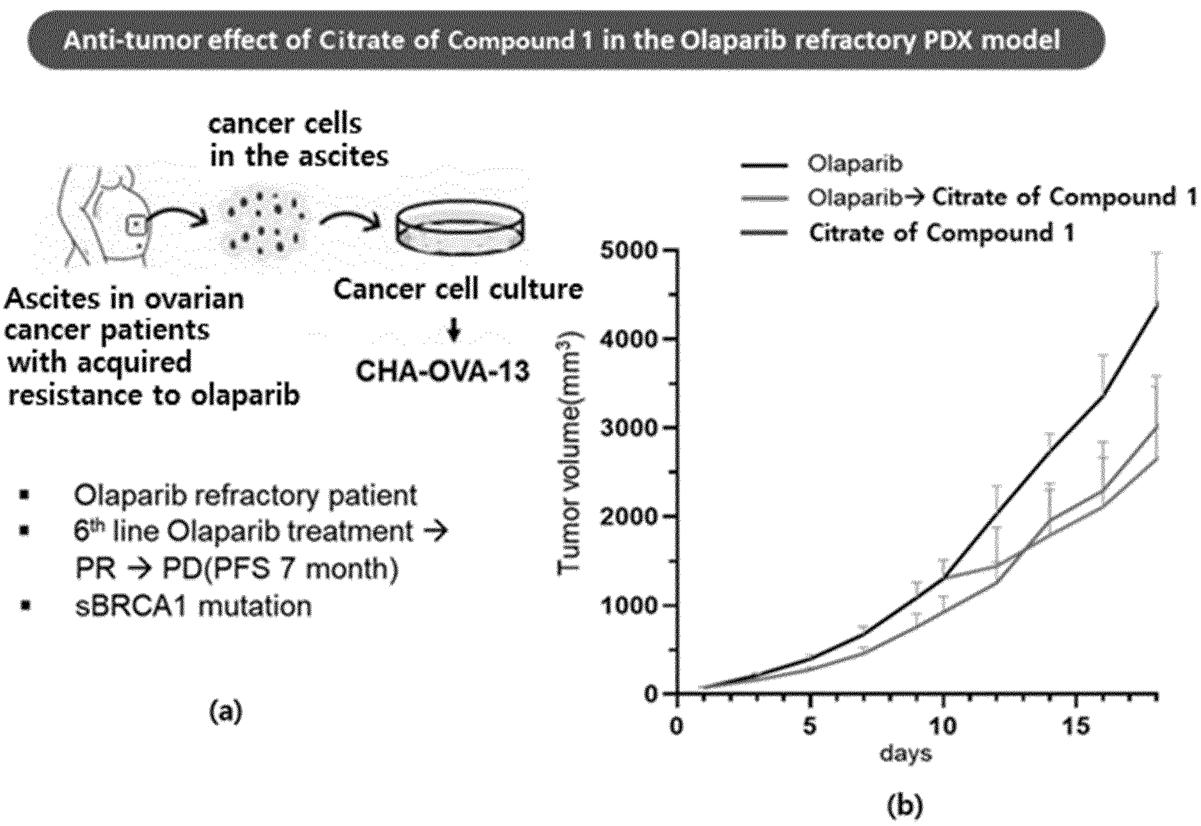
Anti-tumor effect of Citrate of Compound 1 in the Olaparib refractory PDX model
cancer cells
in the ascites
Ascites in ovarian
cancer patients
with acquired
resistance to olaparib
Cancer cell culture
CHA-OVA-13
Olaparib refractory patient
6th line Olaparib treatment →
PR → PD(PFS 7 month)
sBRCA1 mutation
(a)
Olaparib
Olaparib→ Citrate of Compound 1
Citrate of Compound 1
Tumor volume(mm3)
5000
4000
3000
2000
1000
0
0
5
10
15
days
(b)

## INTERNATIONAL SEARCH REPORT

International application No.
**PCT/KR2022/007115**

### A. CLASSIFICATION OF SUBJECT MATTER

**A61K 31/496**(2006.01)i; **A61P 35/00**(2006.01)i

According to International Patent Classification (IPC) or to both national classification and IPC

### B. FIELDS SEARCHED

Minimum documentation searched (classification system followed by classification symbols)

A61K 31/496(2006.01); A61K 31/444(2006.01); A61K 31/502(2006.01); A61K 31/7072(2006.01); C07D 401/12(2006.01); C07D 403/14(2006.01); C07D 471/04(2006.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 6-{4-[5-옥소-1,2,3,4,5,6-헥사히드로벤조[h][1,6]나프티리딘-8-일]메틸}피페라진-1-일}니코티노니트릴(6-{4-[(5-oxo-1,2,3,4,5,6-hexahydrobenzo[h][1,6]naphthyridin-8-yl]methyl}piperazin-1-yl}nicotinonitrile), 폴리(ADP-리보스)폴리머라제(Poly(ADP-Ribose)Polymerase, PARP), 상동 재조합 결핍(homologous recombination deficiency), 유방암 감수성 유전자(breast cancer susceptibility gene, BRCA)

### C. DOCUMENTS CONSIDERED TO BE RELEVANT

| Category* | Citation of document, with indication, where appropriate, of the relevant passages | Relevant to claim No. |
|---|---|---|
| A | KR 10-2016-0021845 A (SHANGHAI HUILUN LIFE SCIENCE & TECHNOLOGY CO., LTD.) 26 February 2016 (2016-02-26)<br>See claims 1, 6, 13 and 73-76; and paragraph [0083]. | 1-14,16 |
| A | KR 10-2017-0005627 A (THE ASAN FOUNDATION) 16 January 2017 (2017-01-16)<br>See claim 1; and paragraph [0045]. | 1-14,16 |
| A | WO 2021-048235 A1 (THE FRANCIS CRICK INSTITUTE LIMITED) 18 March 2021 (2021-03-18)<br>See claim 1. | 1-14,16 |
| A | KR 10-2020-0096788 A (JIANGSU HENGRUI MEDICINE CO., LTD.) 13 August 2020 (2020-08-13)<br>See entire document. | 1-14,16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 August 2022** | **25 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2022/007115**

**C. DOCUMENTS CONSIDERED TO BE RELEVANT**

| Category* | Citation of document, with indication, where appropriate, of the relevant passages | Relevant to claim No. |
|---|---|---|
| A | WO 2021-013735 A1 (ASTRAZENECA AB) 28 January 2021 (2021-01-28)<br>See claims 1, 19 and 21. | 1-14,16 |
| PX | IM, Seock-Ah et al. A phase I dose-escalation and expansion study of JPI-547, a dual inhibitor of PARP/ tankyrase in patients with advanced solid tumors. Journal of Clinical Oncology. 2021, vol. 39, no. 15, p. 3113 (online publication date: 28 May 2021).<br>See page 3113. | 1-14,16 |

Form PCT/ISA/210 (second sheet) (July 2019)

**INTERNATIONAL SEARCH REPORT**

International application No.
**PCT/KR2022/007115**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Claim 15 pertains to a method for treatment of the human body by surgery or therapy, as well as a diagnostic method (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2022/007115**

| Patent document cited in search report | Publication date (day/month/year) | Patent family member(s) | Publication date (day/month/year) |
|---|---|---|---|
| KR 10-2016-0021845 A | 26 February 2016 | AU 2014-283879 A1 | 24 December 2014 |
| | | AU 2014-283879 A1 | 21 January 2016 |
| | | AU 2014-283879 B2 | 08 June 2017 |
| | | CA 2915319 A1 | 24 December 2014 |
| | | CA 2915319 C | 03 October 2017 |
| | | CN 104230896 A | 24 December 2014 |
| | | CN 104981468 A | 14 October 2015 |
| | | CN 104981468 B | 05 January 2018 |
| | | EP 3012256 A1 | 27 April 2016 |
| | | EP 3012256 B1 | 08 August 2018 |
| | | ES 2688191 T3 | 31 October 2018 |
| | | JP 2016-521758 A | 25 July 2016 |
| | | JP 6263614 B2 | 17 January 2018 |
| | | KR 10-2021-0155826 A | 23 December 2021 |
| | | RU 2015153319 A | 24 July 2017 |
| | | RU 2649002 C2 | 29 March 2018 |
| | | US 10196381 B2 | 05 February 2019 |
| | | US 2016-0159776 A1 | 09 June 2016 |
| | | WO 2014-201972 A1 | 24 December 2014 |
| KR 10-2017-0005627 A | 16 January 2017 | CN 107922976 A | 17 April 2018 |
| | | EP 3321377 A1 | 16 May 2018 |
| | | EP 3321377 B1 | 29 December 2021 |
| | | KR 10-1775356 B1 | 06 September 2017 |
| | | US 10683553 B2 | 16 June 2020 |
| | | US 2018-0327851 A1 | 15 November 2018 |
| | | US 2020-0354794 A1 | 12 November 2020 |
| | | WO 2017-007241 A1 | 12 January 2017 |
| WO 2021-048235 A1 | 18 March 2021 | AU 2020-344145 A1 | 18 March 2021 |
| | | GB 201913030 D0 | 23 October 2019 |
| | | KR 10-2022-0064380 A | 18 May 2022 |
| KR 10-2020-0096788 A | 13 August 2020 | AU 2018-380174 A1 | 21 May 2020 |
| | | AU 2018-380174 A1 | 13 June 2019 |
| | | BR 112020010435 A2 | 24 November 2020 |
| | | CA 3080644 A1 | 13 June 2019 |
| | | CN 111093706 A | 01 May 2020 |
| | | CN 113768933 A | 10 December 2021 |
| | | EP 3721906 A1 | 14 October 2020 |
| | | JP 2021-505548 A | 18 February 2021 |
| | | TW 201924720 A | 01 July 2019 |
| | | US 2020-0281923 A1 | 10 September 2020 |
| | | WO 2019-109938 A1 | 13 June 2019 |
| WO 2021-013735 A1 | 28 January 2021 | AU 2020-318599 A1 | 28 January 2021 |
| | | CN 114144413 A | 04 March 2022 |
| | | EP 3999506 A1 | 25 May 2022 |
| | | IL 289534 A | 01 March 2022 |
| | | KR 10-2022-0035941 A | 22 March 2022 |
| | | TW 202116750 A | 01 May 2021 |
| | | US 2021-040084 A1 | 11 February 2021 |
| | | UY 38793 A | 26 February 2021 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210064278
- KR 101136702
- KR 101146806
- KR 101837047
- KR 1020210064416